# EUROPEAN PATENT SPECIFICATION

(11) **EP 1 006 864 B1**
(45) Date of publication and mention of the grant of the patent: **15.10.2003**
(21) Application number: 97906324.5
(22) Date of filing: 21.03.1997
(51) Int. Cl.: A61B 5/00

(54) **METHOD FOR HARMONICALLY FILTERING DATA**
VERFAHREN FÜR DAS HARMONISCHE FILTERN VON DATEN
PROCEDE DE FILTRAGE HARMONIQUE DE DONNEES

(43) Date of publication of application: 14.06.2000
(73) Proprietor: Nellcor Puritan Bennett Incorporated, Pleasanton, CA 94588 (US)
(72) Inventor: BAKER, Clark, R., Jr., Castro Valley, CA 94546 (US); YORKEY, Thomas, J., San Ramon, CA 94583 (US)
(74) Representative: Belcher, Simon James
(86) International application number: IB9700293
(87) International publication number: WO98042251

(56) References cited:
- EP-A- 0 303 502
- WO-A-92/15955
- WO-A-94/09698
- US-A- 4 960 126

## Description

This invention relates to a method for measuring physiological parameters, in particular for reducing noise effects in a system for measuring a physiological parameter. It relates in particular to a method for harmonically filtering data. The invention employs filtering techniques in pulse oximetry to estimate the oxygen saturation of haemoglobin in arterial blood.

Pulse oximeters typically measure and display various blood flow characteristics including but not limited to the oxygen saturation of haemoglobin in arterial blood. Oximeters pass light through blood perfused tissue such as a finger or an ear, and photoelectrically sense the absorption of light in the tissue. The amount of light absorbed is then used to calculate the amount of the blood constituent (e.g., oxyhaemoglobin) being measured.

The light passed through the tissue is selected to be of one or more wavelengths that are absorbed by the blood in an amount representative of the amount of the blood constituent present in the blood. The amount of light passed through the tissue varies in accordance with the changing amount of blood constituent in the tissue and the related light absorption. The calculation of saturation can then be based on Beer-Lambert's law. Traditionally, the determination of saturation measures light absorption at two wavelengths, for example red and infra red. Saturation can then calculated by solving for the "ratio of ratios", as known, for example from US-4928692, US-4934372 and US-5078136.

The optical signal through the tissue can be degraded by both noise and motion artifact. One source of noise is ambient light which reaches the light detector. Another source of noise is electromagnetic coupling from other electronic instruments. Motion of the patient also introduces noise and affects the signal. For example, the contact between the detector and the skin, or the emitter and the skin, can be temporarily disrupted when motion causes either to move away from the skin. In addition, since blood is a fluid, it responds differently than the surrounding tissue to inertial effects, thus resulting in momentary changes in volume at the point to which the oximeter probe is attached.

Motion artifact can degrade a pulse oximetry signal relied upon by a physician, without the physician's awareness. This is especially true if the monitoring of the patient is remote, the motion is too small to be observed, or the doctor is watching the instrument or other parts of the patient, and not the sensor site.

An oximeter system is disclosed in US-5025791 in which an accelerometer is used to detect motion. When motion is detected, readings influenced by motion are either eliminated or indicated as being corrupted. In a typical oximeter, measurements taken at the peaks and valleys of the blood pulse signal are used to calculate the desired characteristic. Motion can cause a false peak, resulting in a measurement having an inaccurate value and one which is recorded at the wrong time.

Another system is disclosed in US-4802486 in which an EKG signal is monitored and correlated to the oximeter reading to provide synchronization to limit the effect of noise and motion artifact pulses on the oximeter readings. This reduces the chances of the oximeter locking onto a periodic motion signal.

The system disclosed in US-5078136 involves the use of linear interpolation and rate of change techniques to analyze the oximeter signal, to limit the effect of noise and motion artifact.

EP-A-0 303 502 discloses a method comprising the features of the generic clause of claim 1 and also step (a).

The present invention provides a technique for measuring a blood constituent value which involves filtering data such that motion and noise energy not an integer multiples of a heart rate of the patient are attenuated, and then assigning variable weights to the filtered data and averaging the resulting data.

Accordingly, the invention provides a method for measuring a blood constituent using data corresponding to a wavelength of electromagnetic energy transmitted through tissue of a patient, the method comprising the steps of:
(a) filtering the data such that motion and noise energy not at integer multiples of a heart rate of the patient are attenuated, thereby generating filtered data;
(b) comparing selected filtered data with at least one expected data characteristic;
(c) assigning one of a plurality of variable weights to each selected filtered data based on the comparing step thereby generating a plurality of differently weighted filtered data, the variable weights comprising a plurality of different non-zero numbers; and
(d) averaging a plurality of the differently weighted filtered data to obtain a twice-filtered data for use in estimating the blood constituent.

Preferably, the filter is characterized by a filter response which varies with the heart rate of the patient.

Preferably, there are a plurality of wavelengths of electromagnetic energy transmitted through the tissue of the patient, the steps of filtering and comparing being applied to data corresponding to only one of the plurality of wavelengths, the steps of assigning and averaging being applied to data corresponding to the plurality of wavelengths based on the filtering and comparing steps being applied to one of the plurality of wavelengths.

The invention will now be described, by way of example only, with reference to the accompanying drawings, in which:
Figure 1 is a schematic representation of apparatus for measuring a physiological parameter such as oxygen saturation of haemoglobin of a patient;
Figure 2 is a block diagram illustrating the flow of data in apparatus such as that shown in Figure 1;

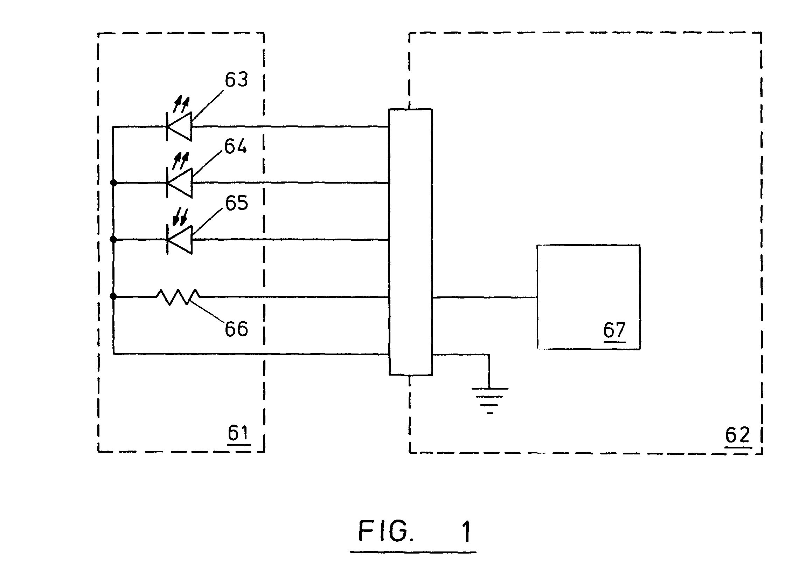

Referring to the drawings, Figure 1 shows apparatus for measuring physiological parameters such as oxygen saturation of haemoglobin of a patient. A sensor/oximeter combination 60 comprises a sensor 61 and an oximeter monitor 62. Sensor 61 includes LEDs 63 and 64 typically having wavelength emission characteristics in the infrared and red ranges of the spectrum, respectively. Photodiode sensor 65 receives the light transmitted by LEDs 63 and 64. Resistor 66 (or a similar electrical impedance reference) is chosen to correspond to a specific wavelength or combination of wavelengths as specified by a table relating impedance values to wavelengths. Decoding means 67 determines the impedance value of resistor 66, and appropriate extinction coefficients are generated which correspond to the transmission characteristics of the particular sensor 61. Thus, the oximeter may be used with a variety of sensors having LEDs which emit varying wavelengths of light without recalibration. The sensor 61 is detachably coupled to oximeter monitor 62 via connector 68. An example of such a sensor/oximeter combination is disclosed in US-4621643.

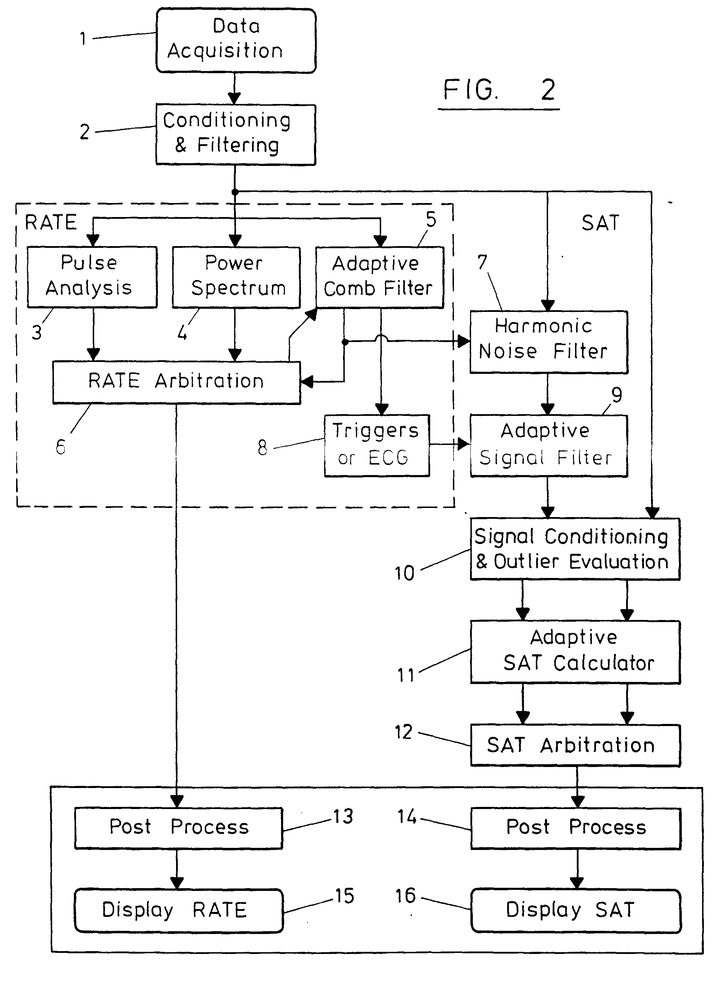

The data received from the sensor is processed according to the scheme shown in Figure 2. It can be processed using apparatus of the type disclosed in US-5348004. In initial process steps 12, 14, the natural logarithm of the data (usually from red and infra red LEDs) is taken (step 12), and the data is band pass filtered with an infinite impulse response (IIR) filter that has a high pass cutoff frequency at 0.5 Hz (that is 30 beats per minute) and a low pass rolloff from 10 to 20 Hz (step 14). The filtered data can then processed by algorithms for calculation of oxygen saturation. The algorithms for processing the filtered data can make use of Kalman filtering, with and without cardiac gated averaging.

Information to be processed by an algorithm such as one which involves Kalman filtering is preferably first processed by a harmonic filter 17. That filter digitally filters the IR and red waveforms such that only energy at integer multiples of the heart rate is allowed through the filter. The response of harmonic filter 17 varies with the pulse rate which is supplied by a pulse rate calculator to attenuate motion and noise energy not at the heart rate. It can be appropriate for just only one of the IR and red waveforms to be filtered by the harmonic filter 17, although frequently, both will be filtered.

Data from the harmonic filter 17 can then be processed by a Kalman filter with cardiac gated averaging, using triggers from an ECG waveform or from an algorithm for calculating pulse rate.

An oxygen saturation value is obtained by application of a second Kalman filter. In contrast to the first Kalman filter with cardiac gated averaging which is event based, the second filter is time based. The second filter operates on data from the band pass filter and on data from the first filter. Prior to application of the second filter, data points resulting in an impossible saturation calculation (for example negative saturation) are rejected. After application of the second filter, the best saturation value is chosen according to confidence levels associated with each value.

The saturation value after the second filter is displayed after appropriate post-processing to determine whether and how it is to be displayed. Confidence levels in the oxygen saturation can be estimated from metrics available from the algorithms performed on the oximeter data, determining which saturation can be considered reliable. For example, the confidence level can be determined dependent on the age of the signal from which the saturation level is calculated and the deviation of that level from an estimated value.

## Claims

1. A method for measuring a blood constituent using data corresponding to a wavelength of electromagnetic energy transmitted through tissue of a patient, the method comprising the steps of:
(a) filtering the data such that motion and noise energy not at integer multiples of a heart rate of the patient are attenuated, thereby generating filtered data;
(b) comparing selected filtered data with at least one expected data characteristic;
(c) assigning one of a plurality of variable weights to each selected filtered data based on the comparing step thereby generating a plurality of differently weighted filtered data, the variable weights comprising a plurality of different non-zero numbers; and
(d) averaging a plurality of the differently weighted filtered data to obtain a twice-filtered data for use in estimating the blood constituent.

2. A method as claimed in claim 1, in which the filter is **characterized by** a filter response which varies with the heart rate of the patient.

3. A method as claimed in claim 1, in which there are a plurality of wavelengths of electromagnetic energy transmitted through the tissue of the patient, the steps of filtering and comparing being applied to data corresponding to only one of the plurality of wavelengths, the steps of assigning and averaging being applied to data corresponding to the plurality of wavelengths based on the filtering and comparing steps being applied to one of the plurality of wavelengths.

## Patentansprüche

1. Verfahren zum Messen eines Blutbestandteiles unter Verwendung von Daten, die einer Wellenlänge elektromagnetischer Energie entsprechen, welche durch das Gewebe eines Patienten übertragen wird, wobei das Verfahren die Schritte umfasst:
(a) Filtern der Daten, so dass Bewegung und Rauschenergie, die nicht bei ganzzahligen Vielfachen einer Herzfrequenz des Patienten liegen, gedämpft werden, wodurch gefilterte Daten erzeugt werden;
(b) Vergleichen von ausgewählten gefilterten Daten mit mindestens einer erwarteten Datencharakteristik;
(c) Zuordnen eines einer Mehrzahl von variablen Gewichten zu jedem ausgewählten gefilterten Datenwert auf der Basis des Vergleichsschrittes, wodurch eine Mehrzahl von verschieden gewichteten gefilterten Daten erzeugt wird, wobei die variablen Gewichte eine Mehrzahl von verschiedenen Zahlen ungleich Null umfassen; und
(d) Bilden des Durchschnitts einer Mehrzahl der verschieden gewichteten gefilterten Daten, um zweimal gefilterte Daten zur Verwendung bei der Abschätzung des Blutbestandteils zu erhalten.

2. Verfahren nach Anspruch 1, in dem das Filter durch einen Filterfrequenzgang charakterisiert ist, der mit der Herzfrequenz des Patienten variiert.

3. Verfahren nach Anspruch 1, in dem eine Mehrzahl von Wellenlängen elektromagnetischer Energie vorliegt, welche durch das Gewebe des Patienten übertragen wird, wobei die Schritte des Filterns und des Vergleichens auf Daten angewendet werden, die nur einer der Mehrzahl von Wellenlängen entsprechen, wobei die Schritte der Zuordnung und der Bildung des Durchschnitts auf Daten, welche der Mehrzahl von Wellenlängen entsprechen, auf der Basis des Filterns angewendet werden und Vergleichsschritte auf eine der Mehrzahl von Wellenlängen angewendet werden.

## Revendications

1. Procédé pour mesurer un constituant du sang en utilisant des données correspondant à une longueur d'onde d'une énergie électromagnétique transmise par le tissu d'un patient, le procédé comprenant les étapes consistant à:
(a) filtrer les données de telle sorte que l'énergie de mouvement ou déplacement et l'énergie de bruit, qui ne sont pas des multiples entiers d'un rythme cardiaque du patient, sont atténuées, ce qui permet d'obtenir des données filtrées;
(b) comparer des données filtrées sélectionnées à au moins une caractéristique de données attendue;
(c) affecter l'un d'une pluralité de poids variables à chaque donnée filtrée sélectionnée sur la base de l'étape de comparaison en produisant ainsi une pluralité de données filtrées pondérées différemment, les poids variables comprenant une pluralité de nombres non nuls différents; et
(d) former la moyenne de la pluralité des données filtrées pondérées différemment pour obtenir des données filtrées deux fois destinées à être utilisées pour estimer le constituant du sang.

2. Procédé selon la revendication 1, dans lequel le filtre est **caractérisé par** une réponse de filtre, qui varie avec le rythme cardiaque du patient.

3. Procédé selon la revendication 1, selon lequel une pluralité de longueurs d'onde d'énergie électromagnétique sont transmises par le tissu du patient, les étapes de filtrage et de comparaison étant appliquées à des données correspondant uniquement à l'une de la pluralité de longueurs d'onde, les étapes d'affectation et de formation de la moyenne étant appliquées à des données correspondant à la pluralité de longueurs d'onde sur la base des étapes de filtrage et de comparaison appliquées à l'une de la pluralité de longueurs d'onde.
